# EUROPÄISCHE PATENTSCHRIFT

(11) **EP 2 827 939 B1**
(45) Veröffentlichungstag und Bekanntmachung des Hinweises auf die Patenterteilung: **29.07.2015**
(21) Anmeldenummer: 12710235.8
(22) Anmeldetag: 20.03.2012
(51) Int. Cl.: A61M 39/24, A61M 5/142

(54) **EINWEGEVENTIL FÜR EIN INFUSIONSBESTECK**
ONE-WAY VALVE FOR AN INFUSION INSTRUMENT
VALVE UNIDIRECTIONNELLE POUR SET DE PERFUSION

(43) Veröffentlichungstag der Anmeldung: 28.01.2015
(73) Patentinhaber: Cedic S.r.l., 20068 Peschiere Borromeo (MI) (IT)
(72) Erfinder: MIJERS, Jan Willem Marinus, 2014 AL Haarlem (NL)
(74) Vertreter: Caspary, Karsten
(86) Internationale Anmeldenummer: PCT/EP2012/054875
(87) Internationale Veröffentlichungsnummer: WO 2013/139374

(56) Entgegenhaltungen:
- WO-A1-03/018105
- FR-A1- 2 910 817
- US-A- 6 036 171
- US-A1- 2002 117 645

## Beschreibung

Die Erfindung betrifft ein Einwegeventil, insbesondere für medizinische Anwendung, umfassend zumindest ein Ventilgehäuse, eine Einlass- und Auslassöffnung und ein Ventilanordnung, welche aus einem Ventilkörper und einer Ventildichtung besteht.

Gattungsgemäße Einwegeventile für die Medizintechnik werden beispielsweise für ein Infusionsbesteck benötigt. Hierbei kann es sich um ein Infusionsbesteck handeln, das beispielsweise mittels der Schwerkraft zunächst befüllt wird und in der Regel aus einem Flüssigkeitsbehälter besteht, welcher gegenüber einem Patienten erhöht gelagert wird. Über Schlauchverbindungen und eine Injektionsnadel wird die Infusionslösung dem Patienten zugeführt, wobei mit Hilfe einer Schlauchklemme der Flüssigkeitsstrom einstellbar ist. Mit Hilfe von Abzweigungen, beispielsweise in T- oder Y-Form, kann eine Infusionspumpe angeschlossen werden, welche beispielsweise dazu verwendet wird, um in regelmäßigen Zeitabständen ein flüssiges Medikament dem Patienten zuzuführen. Ähnlich verhält es sich bei der Zuführung von Nahrungsmittellösungen über eine Magensonde. Auch hier wird dem Patienten aus einem Vorratsbehälter die Nahrungsmittellösung über einen Schlauch zur Magensonde zugeführt, wobei zusätzlich mit Hilfe einer Infusionspumpe in regelmäßigen Zeitabständen flüssige Medikamente zugeführt werden können oder ein Transport der Nahrungsmittellösung über eine Pumpe, vorzugsweise Schlauchpumpe gefördert wird. Bei einer Schlauchpumpe besteht im einfachsten Fall die Möglichkeit den Schlauch des Infusionsbestecks in eine vorhandene Ausnehmung der Schlauchpumpe einzulegen, sodass die Schlauchpumpe mit Hilfe eines drehbaren Rades durch Zusammenquetschen des Schlauches einen Transport der Flüssigkeit bewirkt. Die Drehrichtung der Schlauchpumpe bestimmt hierbei die Transportrichtung der Flüssigkeit. Derartige Schlauchpumpen eignen sich hervorragend, um beispielsweise Nahrungsmittellösungen mit zum Teil größeren Feststoffen zu transportieren. Aus diesem Grunde werden die Schlauchpumpe in der Regel oberhalb des Einwegeventils angeordnet, um einen entsprechenden Förderdruck zu erzeugen. Bei dieser Anordnung muss jedoch sichergestellt sein, dass keine Flüssigkeit aus einem möglicherweise offenen Schlauchende des Infusionsbestecks austreten kann (Antisiphon-Lösung) und der Förderdruck ausreichend ist, um eine kontinuierliche und unterbrechungsfreie Zuführung der Nahrungsmittellösung zum Patienten zu gewährleisten. Gegenüber reinen Infusionslösungen weisen die Nahrungsmittellösungen zusätzliche Ballaststoffe auf, welche eine andere Konsistenz aufweisen und somit zu einem Verstopfen des Einwegeventils führen können, beziehungsweise die dort vorhandenen Durchtrittsöffnungen blockieren können, sodass das Einwegeventil seine normale Funktion nicht erfüllen kann. Um zu verhindern, dass die Infusionslösung und/oder das Medikament aus dem Infusionsbesteck aufgrund der Schwerkraft austreten kann, werden Einwegeventile eingesetzt, die einerseits einen notwendigen Öffnungsdruck erfordern und andererseits im Falle einer Spülung des Infusionsbestecks unterhalb des Einwegeventils einen Rückfluss in Richtung des Infusionsbeutel verhindern. Eine Spülung ist hierbei nach jeder Nahrungsmittelzuführung erforderlich, um die Bildung von Bakterien im Infusionsbesteck zu verhindern. Zu diesem Zweck befindet sich unterhalb des Einwegeventils eine T- oder Y-Abzweigung, an die eine mit Wasser gefüllte Spritze angeschlossen werden kann.

Darüber hinaus besteht sowohl bei Infusionslösungen als auch bei Nahrungsmittellösungen ein Problem dadurch, dass ein Befüllen des gesamten Infusionsbestecks recht zeitaufwendig ist, weil eine Befüllung der vorhandenen Zuleitungen mittels der Schwerkraft erfolgen muss.

Ein derartiges Einwegeventil, wie es in dem Oberbegriff nach Anspruch 1 beschrieben ist, ist auch aus dem Dokument WO03/018105 A1 bekannt.

Aus der US-Anmeldung 2007/0246674 ist ein Ventil für medizinische Zwecke bekannt, welches aus zwei Ventilgehäuseteilen und einem Kolben besteht. In den verschiedenen Ausführungsformen kann der Kolben durch Druckbeaufschlagung axial innerhalb der beiden Gehäuseteile bewegt werden, wobei eine Abdichtung des Kolbens durch Dichtungselemente, zum Beispiel in Form von O-Ringen, erfolgt. Zusätzlich erfolgt zwischen dem Kolben und den beiden Gehäuseteilen eine Abdichtung durch eine Membran, welche jeweils endseitig zwischen den beiden Gehäuseteilen beziehungsweise einem Gehäuseteil und dem Kolben einklemmend gehalten ist. Durch die Axialbewegung des Kolbens wird hierbei das Dichtungselement dazu verwendet, einen vorhandenen Flüssigkeitsraum in der Größe zu verändern.

Der Erfindung liegt daher die Aufgabe zugrunde, ein Einwegeventil aufzuzeigen, welches die aus dem Stand der Technik bekannten Probleme vermeidet.

Erfindungsgemäß ist zur Lösung der Aufgabe vorgesehen, dass der Ventilkörper eine Durchlassöffnung aufweist, welche in einem abdichtbaren Freiraum zwischen dem Ventilkörper und der Ventildichtung mündet, und dass die Ventildichtung eine Öffnung aufweist, welche durch den Ventilkörper bei einer Relativbewegung zweier Gehäuseteile derart verschließbar ist, dass die Ventildichtung mit einer Dichtungslippe an dem Ventilkörper elastisch anhebbar anliegt. Weitere vorteilhafte Ausgestaltungen ergeben sich aus den Unteransprüchen.

Die erfindungsgemäße Lösung zur Ausbildung eines Ventilkörpers mit einer zusätzlichen Durchlassöffnung ermöglicht ein wesentlich schnelleres Befüllen des gesamten Leitungssystem eines Infusionsbesteckes, sodass beispielsweise eine Krankenschwester nach Anschluss der Infusionslösung oder des Nahrungsmittelbehälters durch Öffnen einer Schlauchklemme das gesamte Leitungssystem befüllt und anschließend durch einen manuellen Druck von außen die vorhandene großvolumige Durchlassöffnung verschließen kann. Dieser Vorgang ist nur einmalig beim Anschluss der Infusionslösung beziehungsweise der Nahrungsmittellösung erforderlich. Nach erfolgtem Verschließen der Durchlassöffnung erfüllt das Einwegeventil seine normale Funktion, sodass die Infusionslösung oder die Nahrungsmittellösung über einen vorhandenen Ventilkörper mit Ventildichtung geleitet wird, wobei wesentlich geringere Querschnitte zugrunde liegen, um den Flüssigkeitstransport mit Hilfe der Pumpe im vorgesehenen Umfang vornehmen zu können. Der wesentliche Vorteil bei dieser Lösung besteht darin, dass die Befüllung und Handhabung des Infusionsbestecks vereinfacht und beschleunigt wird und somit beispielsweise eine Krankenschwester deutlich weniger Zeit benötigt. Nachdem die Öffnung verschlossen wurde, besteht nur die Möglichkeit mit Hilfe einer Infusions- oder Schlauchpumpe einen notwendigen Förderdruck aufzubauen, damit die Dichtungslippe von dem Ventilkörper abheben kann, gleichzeitig wird sichergestellt, dass im Falle eines Rückstaus auf der Ventilausgangsseite mit einem entstehenden Überdruck die Dichtungslippe an den Ventilkörper gepresst wird, sodass ein Zurückdrücken der Infusionslösung oder der Spüllösung verhindert wird. Dies ist beispielsweise der Fall, wenn der untere Bereich des Infusionsbestecks zum Patienten hin gespült werden muss.

Ein weiterer Vorteil des erfindungsgemäßen Einwegeventils besteht dadurch, dass nach dem Verschließen der Durchgangsöffnung ein Tropfen des Infusionsbestecks verhindert wird und erst ein angemessener Förderdruck eine Öffnung der Ventildichtung bewirkt.

Als besonders vorteilhaft hat sich hierbei herausgestellt, dass zur Aufrechterhaltung der normalen Funktion des Einwegeventils ein Ventilkörper verwendet wird, welcher zur Anlagerung einer Dichtungslippe vorgesehen ist, wobei die Ventildichtung zum Ventilkörper beabstandet angeordnet und ein Freiraum ausgebildet ist. Der Freiraum dient zum Befüllen mit der Infusions- oder Nahrungsmittellösung, sodass ein relativ großvolumiger Öffnungsquerschnitt geschaffen wird und eine leichte Befüllung aufgrund der Schwerkraft möglich ist. Als bevorzugte Ausführungsvariante wird ein Einwegeventil vorgeschlagen, welches sowohl eine Durchlassöffnung zur Erstbefüllung als auch einen im Querschnitt großvolumigen Freiraum zwischen dem Ventilkörper und der Ventildichtung aufweist, damit auch im Falle der Zuführung einer Nahrungsmittellösung mit Ballaststoffen durch deren Korngröße kein Verstopfen des Einwegeventils eintritt und ein sicheres Schließen der Dichtungslippe an dem Ventilkörper gewährleistet ist. Durch die besondere Auswahl einer Dichtungslippe unter Vermeidung einer flächenmäßigen Anlage des Dichtungselementes an dem Ventilkörper können somit auch größere Partikel wie Ballaststoffe ohne Probleme den vorhandenen Dichtungsabschnitt der Dichtungslippe gegenüber dem Ventilkörper bei entsprechendem Förderdruck durchdringen und führen insbesondere nicht dazu, dass die Ballaststoffe zwischen der Dichtungslippe und dem Ventilkörper eingeklemmt werden, sodass die Funktion des Einwegeventils beeinträchtigt wäre.

In Ausgestaltung der Erfindung ist vorgesehen, dass das Ventilgehäuse aus zumindest zwei Ventilgehäuseteilen besteht, welche wenigstens teilweise ineinander- beziehungsweise zusammenschiebbar ausgebildet sind. Die zusammenschiebbare Lösung wird immer dann eingesetzt, wenn das Einwegeventil zunächst zur schnellen Befüllung mittels Schwerkraft eingesetzt werden soll, wobei die Durchlassöffnung zunächst geöffnet ist und durch Zusammendrücken der beiden Ventilgehäuseteile verschlossen wird. Durch die Relativbewegungen zwischen den beiden Ventilgehäuseteilen erfolgt somit ein Verschluss der Durchlassöffnung durch einen korrespondierenden Ventilkörper. Das Ventilgehäuse selbst ist rotationssymmetrisch und axial bauend ausgebildet, gleichbedeutend mit einer länglichen Erstreckung, wobei endseitig die Schlauchleitungen anschließbar sind. Darüber hinaus ist denkbar, dass das Ventilgehäuse teilweise rotationssymmetrisch ausgebildet ist und zusätzlich abgewinkelt ausgeführt wird, beispielsweise in einem rechten Winkel, sodass die obere Zuleitung unmittelbar an einem rotationssymmetrischen Ventilgehäuseteil angeschlossen wird, während eine weitere Schlauchleitung an dem zweiten Ventilgehäuseteil, welches abgewinkelt ausgeführt ist, anschließbar ist.

In Ausgestaltung der Erfindung ist vorgesehen, dass ein erstes Ventilgehäuseteil mit einem zentrisch angeordneten Ventilkörper einstückig verbunden ist. Der Ventilkörper befindet sich innerhalb eines ersten Ventilgehäuseteils, wobei in besonderer Ausgestaltung der Ventilkörper über Radialstege mit dem Ventilgehäuseteil verbunden ist, damit zwischen den Radialstegen ausreichend groß dimensioniert Öffnungen ausgebildet sind, durch die die Flüssigkeit hindurchtreten kann.

In weiterer Ausgestaltung besitzt der Ventilkörper eine Einlassöffnung, welche in die Durchlassöffnung mündet und eine Auslassöffnung unterhalb der Ventilöffnung. Zwischen Einlass- und Auslassöffnung befinden sich der Ventilkörper und die Ventildichtung, welche an die Ventilkörper nach dem Zusammendrucken der beiden Ventilgehäuseteile anliegt. Der Ventilkörper besteht hierbei aus einem Kunststoffteil, vorzugsweise aus einem einstückig mit einem Ventilgehäuseteil verbundenen Kunststoffteil. Die Ventildichtung besteht aus einem elastischen Material, welches an den Ventilkörper anliegt und aufgrund der Elastizität bei einem Überdruck abheben kann. Zum Verschließen der Öffnung wird beim Zusammendrücken der beiden Ventilgehäuseteile der Ventilkörper in die Ventildichtung gepresst, sodass die Dichtungslippe der Ventildichtung an einer konischen Anschlagfläche des Ventilkörpers zu liegen kommt. Der Ventilkörper kann hierbei über Radialstege mit dem Ventilgehäuseteil verbunden sein. Es besteht aber bei einer abgewinkelten Ausführung die Möglichkeit, dass der Ventilkörper aus einer Radialfläche herausragt. In jedem Fall ist hierbei sichergestellt, dass nach Verschließen der Durchlassöffnung innerhalb der beiden Ventilgehäuseteile die durch die Dichtungslippen hindurchtretenden Flüssigkeiten in die Auslassöffnung gelangen. Hierbei liegt die Dichtungslippe linienförmig an dem Ventilkörper elastisch anhebbar an, sodass auch größere Körner der Ballaststoffe durch einen Förderdruck durch die Ventildichtung gelangen können.

Damit die beiden Ventilgehäuseteile zusammengedrückt werden können, um die Durchlassöffnung zu verschließen weist ein Ventilgehäuseteil eine Druckfläche, beispielsweise für einen Daumen auf, während das zweite Ventilgehäuseteil eine Gegenhalterfläche aufweist. Die Gegenhalterfläche kann beispielsweise aus einem Flanschkragen oder einer Radialfläche bestehen, die zur Anlage des Zeigefingers und Mittelfingers vorgesehen ist, sodass mit Hilfe des Daumens das Einwegeventil zusammengedrückt werden kann, um die Durchflussöffnung nach Befüllung zu verschließen und danach nur die Funktion des Einwegeventils aufrecht zu erhalten.

Zur Vormontage und zur Fixierung der beiden Gehäuseteile zueinander nach dem der entsprechende Anpressdruck aufgebracht wurde, besitzt in weiterer Ausgestaltung der Erfindung ein Gehäuseteil ein oder mehrere Raststufen auf einer Außenfläche auf, während das korrespondierende Ventilgehäuseteil ein oder mehrere umfangsverteilte Rastnasen aufweist. Die Rastnasen dienen hierbei zum Zusammenhalt der beiden Ventilgehäuseteile und können aus einer ersten Raststufe, die bei der Vormontage vorgegeben wird, in eine weitere Raststufe gleiten, wobei vorzugsweise scharfkantige Rastnasen verwendet werden, sodass ein sicherer Rückhalt in den jeweiligen Raststufen gewährleistet ist. Die Rastnasen und Raststufen sind auch nur zur einmaligen Verwendung vorgesehen, da eine spätere Öffnung des zusammengedrückten Einwegeventils nicht beabsichtigt ist. Derartige Einwegeventile sind vorzugsweise zum einmaligen Gebrauch bei einem Patienten vorgesehen und müssen aus hygienischen Gründen danach entsorgt werden.

Um ein unbeabsichtigtes Zusammendrücken der beiden Ventilgehäuseteile zu verhindern kann in Ausgestaltung der Erfindung ein Sicherungselement vorgesehen sein, welches in einer vorhandenen Aussparung im Bereich der Raststufen eingesetzt wird, um die beiden Ventilgehäuseteile gegeneinander zu verriegeln. Das Sicherungselement besteht beispielsweise aus einem Kreisringsegment mit Griffhalterung und kann somit von außen auf ein Ventilgehäuse aufgesetzt werden und zwar im Bereich der Raststufen, sodass diese blockiert sind. Nach Entfernung des Sicherungselementes, welches ebenfalls nur zum einmaligen Gebrauch vorgesehen ist, können die beiden Ventilgehäuseteile zusammengedrückt werden.

In weiterer Ausgestaltung der Erfindung ist vorgesehen, dass zwischen den Ventilgehäuseteilen eine Ventildichtung angeordnet ist, welche in einer Eindrehung des ersten Ventilgehäuseteils einliegt und durch eine Anschlagfläche gehalten wird und mit zumindest einer ersten Dichtlippe an dem zweiten Ventilgehäuseteil abdichtend und mit einer weiteren Dichtungslippe an dem Ventilkörper anliegt. Die für die Erfindung vorgesehene Ventildichtung wird gleichzeitig zum Abdichten der beiden Ventilgehäuseteile gegeneinander als auch zum Abdichten gegenüber dem Ventilkörper eingesetzt. Aus diesem Grunde liegt die Ventildichtung in einer Eindrehung eines ersten Ventilgehäuseteils ein und mit zumindest einer ersten Dichtlippe an dem zweiten Ventilgehäuseteil abdichtend an. In der Regel werden die Ventilgehäuseteil entweder zusammengeclipst oder nach der Montage miteinander verschweißt oder bei der oben beschriebenen Lösung über Rastnasen und Raststufen gehalten, sofern eine radiale Verschiebung der beiden Ventilgehäuseteile zu einem späteren Zeitpunkt gewünscht wird. Zur Erhöhung der Dichtigkeit kann diese erste Abdichtung mehrere Dichtlippen aufweisen, welche an einer Axialfläche eines Ventilgehäuses anpressbar anliegen, während die zweite Dichtungslippe an dem Ventilkörper anliegt und elastisch ausgebildet ist, sodass diese von dem Ventilkörper abheben kann, damit die Infusionsflüssigkeit oder Nahrungsmittelflüssigkeit durch die zwischen dem Ventilkörper und der Dichtungslippe gebildeten Ventilöffnung hindurchtreten kann. Ebenso wie die Ventilgehäuseteile rotationssymmetrisch ausgebildet sind, ist auch der Ventilkörper rotationssymmetrisch ausgebildet und einstückig mit einem Ventilgehäuseteil verbunden, wobei zumindest eine Eintrittsöffnung von der Einlassöffnung eines ersten Ventilgehäuses zum Freiraum führt. Hierbei ist es denkbar, dass mehrere Eintrittsöffnungen aufgrund der vorliegenden Befestigung des Ventilkörpers innerhalb des Ventilgehäuses ausgebildet sind, welche alle in den Freiraum münden.

Wenn die beiden Ventilgehäuseteile zusammengedrückt wurden, wird die Strömung der zugeführten Nahrungsmittel oder Infusionslösung unterbrochen. Die Strömungsrichtung führt ausgehend von der Einlassöffnung zu den Eintrittsöffnungen des Ventilkörpers bis zur anliegenden Dichtungslippe an dem Ventilkörper, wobei durch einen Druckaufbau infolge einer Infusions- oder Schlauchpumpe ein Abheben der Dichtungslippe gegenüber dem Ventilkörper eine Verbindung zur Auslassöffnung herstellt. Somit ist durch die Infusionspumpe, welche auf die Nahrungsmittel- oder Infusionslösung einwirkt, ein Öffnen des Ventils möglich. Bei einem sich aufbauenden Gegendruck, beispielsweise bei einem Spülvorgang unterhalb des Einwegeventils erfolgt eine Sperrung, weil durch den entstehenden Überdruck die Ventildichtung gegen den Ventilkörper gedrückt wird. Der prinzipielle Aufbau des Infusionsbestecks sieht hierbei vor, dass die Infusions- oder Schlauchpumpe oberhalb des Einwegeventils eingebaut wird, sodass ein Förderdruck aus dem Infusionsbeutel heraus auf das Einwegeventil zum Öffnen der Dichtungslippe gegenüber dem Dichtungskörper aufgebaut wird. Zu diesem Zweck besteht beispielsweise je nach Bauart der Infusionspumpe die Möglichkeit diese über eine T- oder Y-förmige Abzweigung anzuschließen oder es wird eine Schlauchpumpe verwendet, um einen Teil des Schlauches in eine Führung der Schlauchpumpe einzulegen und durch ein Betätigungsrad, welches unmittelbar auf den Schlauch einwirkt, wird die Förderung der Nahrungsmittel oder Infusionslösung in einer Richtung vorgenommen. Der hierbei entstehende Förderdruck reicht aus, um die Ventildichtung von dem Ventilkörper abheben zu lassen, wobei gleichzeitig infolge einer Rückschlagfunktion des Einwegeventils aufgrund der bestehenden Druckdifferenz oberhalb und unterhalb des Einwegeventils erfüllt ist.

Die verwendete Ventildichtung ist ebenfalls rotationssymmetrisch ausgebildet und umschließt koaxial den Ventilkörper, wobei eine nach innen weisende Dichtungslippe unmittelbar am Ventilkörper elastisch anliegt, sodass die Schließ- und Öffnungsfunktion des Ventils gewährleistet ist. Die Vorspannung, das heißt der Öffnungsdruck dieser Dichtungslippe kann durch den Durchmesser des Ventilkörpers, die Dicke der Dichtungslippe oder den Innendurchmesser der Dichtungslippe bestimmt werden, sodass eine Vorspannung eingestellt werden kann. Alternativ kann zusätzlich über die Shore-Härte des verwendeten Dichtungsmaterials die Elastizität und damit der Öffnungsdruck bestimmt werden, wobei die Dichtungslippe bei einem Druckaufbau von etwa 20 bis etwa 300 kPascal von dem Ventilkörper abhebt, damit die Infusions- oder Nahrungsmittellösung infolge des Förderdruckes durch das Einwegeventil nach unten abfließen kann. Vorzugsweise werden für die Ventildichtung hierbei Dichtungsmaterialien mit einer Shore-Härte von 60 bis 80 SH-A verwendet, sodass ein Mindestdruck erforderlich ist, um die Dichtungslippe vom Dichtungskörper abzuheben. Hierdurch wird sichergestellt, dass ein Nachlaufen oder Tropfen der Infusionslösung aufgrund der Schwerkraft vermieden wird.

Die Erfindung wird im Weiteren anhand der Figuren nochmals erläutert.

Es zeigt
- Fig. 1: in einer perspektivischen geschnittenen Seitenansicht ein erfindungsgemäßes Einwegeventil,
- Fig. 2: in einer Explosionsdarstellung das Einwegeventil gemäß Figur 1 mit sämtlichen Einzelteilen,
- Fig. 3: in einer geschnittenen perspektivischen Seitenansicht ein erstes Ventilgehäuseteil,
- Fig. 4: in einer geschnittenen perspektivischen Seitenansicht ein zweites Ventilgehäuseteil,
- Fig. 5: in einer geschnittenen perspektivischen Seitenansicht die Ventildichtung,
- Fig. 6: in einer perspektivischen Seitenansicht das aus Figur 1 bekannte Einwegeventil mit Sicherungselement,
- Fig. 7: in einer perspektivischen Seitenansicht das Einwegeventil gemäß Figur 6 nach Entfernen des Sicherungselementes,
- Fig. 8: in einer perspektivischen geschnittenen Seitenansicht das Sicherungselement und
- Fig. 9: in einer schematischen Ansicht den Aufbau eines Infusionsbestecks mit Infusionspumpe.

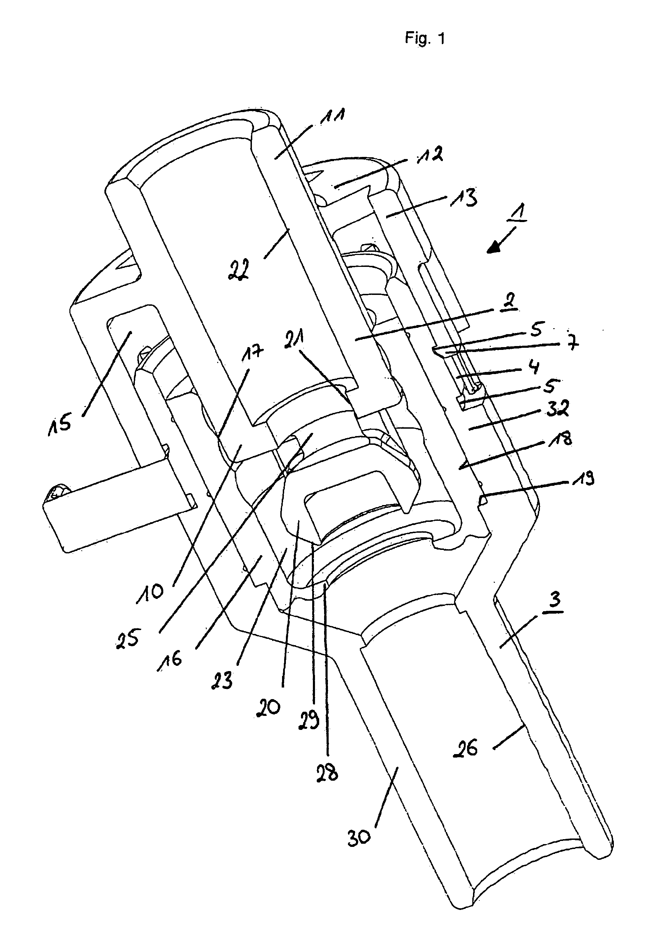

Figur 1 zeigt in einer perspektivischen geschnittenen Seitenansicht ein Einwegeventil 1, welches im gezeigten Ausführungsbeispiel aus einem Ventilgehäuseteil 2 und einem Ventilgehäuseteil 3 besteht. Die beiden Ventilgehäuseteile 2, 3 sind ineinandereinschiebbar ausgebildet, wobei das Ventilgehäuseteil 3 auf einer Außenfläche 4 Raststufen 5 aufweist, in die Rastnasen 7 des weiteren Ventilgehäuseteils 2 eingreifen. Die beiden Ventilgehäuseteile 2, 3 sind insoweit gegeneinanderverschiebbar, wie dies durch die gezeigten Rastnasen 7 und Raststufen 5 axial möglich ist.

Das erste Ventilgehäuseteil 2 besteht aus dem mittleren Gehäuseteil 10, welches nach außen in einen konisch verjüngten Stutzen 11 zum Aufschieben, beispielsweise eines Schlauches, übergeht. Das Gehäuseteil 10 ist über radiale Verbindungsstreben 12 mit dem äußeren Gehäusering 13 verbunden, wobei die Verbindungsstreben 12 über den gesamten Umfang des Ventilgehäuseteils 2 ausgebildet sind. Das obere Ventilgehäuse 2 besitzt eine Stirnfläche 14, welche zum Zusammendrücken der beiden Ventilgehäuseteile 2, 3 verwendbar ist. Auf der Stirnfläche 14 können beispielsweise der Zeige- und Mittelfinger zu diesem Zweck aufgelegt werden. Das Ventilgehäuseteil 2 weist im Weiteren eine Ausnehmung 15 auf, welche durch das Gehäuseteil 10 und den Gehäusering 13 gebildet wird und zur Aufnahme einer Ventildichtung 16 und des zweiten Ventilgehäuseteils 3 vorgesehen ist. Die Ventildichtung 16 liegt mit mehreren Dichtlippen 17 an einer innen liegenden Umfangsfläche des Gehäuseteils 10 abdichtend an. Die Ventildichtung 16 ist hierbei rotationssymmetrisch ausgebildet und besteht aus einem Hohlkörper, welcher im Weiteren in einer Eindrehung 18 des Ventilgehäuseteils 3 einliegt und an einer Anlageschulter 19 abstützend anliegt. Ein rotationssymmetrischer Ventilkörper 20 ist hierbei einstückig mit dem ersten Ventilgehäuseteil 2 unterhalb des Gehäuseteils 10 verbunden. Der Ventilkörper 20 ist über mehrere umfangsverteilte Stege 25 mit dem Ventilgehäuse 2 in derart verbunden, dass zwischen den Stegen 25 Öffnungen 27 entstehen, welche eine Verbindung zu einem Freiraum 23 zwischen dem Ventilkörper 20 und der Ventildichtung 16 herstellen. Dieser Freiraum 23 ist zunächst zur Auslassöffnung 26 hin geöffnet und wird durch Zusammendrücken der beiden Ventilgehäuseteile 2, 3 durch Anlage einer Dichtungslippe 28 an einer konischen Anlagefläche 29 des Ventilkörpers 20 verschlossen, sodass aufgrund der Elastizität der Ventildichtung 16 und der Dichtungslippe 28 die Möglichkeit des Abhebens besteht, um einen Flüssigkeitstransport zu gewährleisten, der bei einer Druckerhöhung auf Seiten der Auslassöffnung 26 zu einer abdichtenden Ventilfunktion führt. Das Ventilgehäuseteil 2 besitzt im gezeigten Ausführungsbeispiel eine Durchlassöffnung 21, welche einenends in die Einlassöffnung 22 mündet und anderenends durch Öffnungen 27 in den Freiraum 23 und danach in die Auslassöffnung 26 mündet. Zwischen dem Ventilkörper 20 und der Ventildichtung 16 ist der Freiraum 23 ausgebildet, welcher einerseits durch die Form des Ventilkörpers 20 und andererseits durch die Form der Ventildichtung 16 begrenzt ist, wobei dieser im unteren Bereich durch die Dichtungslippe 28 verschließbar ist. Im oberen Bereich sind Öffnungen 27 ringförmig unterhalb des mittleren Gehäuseteils 10 des ersten Ventilgehäuseteils 2 ausgebildet, sodass eine eintretende Flüssigkeit durch die Einlassöffnung 22 über die Öffnungen 27 in den Freiraum 23 gelangt und infolge eines Druckaufbaus, beispielsweise durch eine Infusionspumpe, die Dichtungslippe 28 von dem Ventilkörper 20 abgehoben wird und die Flüssigkeit somit unterhalb des Ventilkörpers 20 austreten kann, wenn der Freiraum 23 nach Zusammendrücken der beiden Ventilgehäuseteile 2, 3 verschlossen ist.

Das zweite Ventilgehäuseteil 3 ist ebenfalls rotationssymmetrisch ausgebildet und besteht im Wesentlichen aus einem Hohlkörper mit einem Stutzen 30, der die Auslassöffnung 26 aufweist. Auch der Stutzen 30 ist zum Aufschieben eines Schlauches vorgesehen, sodass das Einwegeventil 1 in eine Schlauchleitung eingesetzt werden kann. Das Ventilgehäuseteil 3 besitzt oberhalb des Stutzens 30 einen sich radial erweiternden Gehäusering 32, welcher auf seiner Außenfläche 4 die Raststufen 5 zur Aufnahme des ersten Ventilgehäuseteils 2 besitzt.

Das erfindungsgemäße Einwegeventil 1 wird in der in Figur 1 dargestellten Form ausgeliefert, und zwar mit einem unverschlossenen Freiraum 23, welcher einenends in die Einlassöffnung 22 über die Durchbrüche 27 mündet und anderenends in die Auslassöffnung 26 mündet. Durch diese Maßnahme ist beispielsweise sichergestellt, dass die Infusionslösung oder eine Nahrungsmittellösung aus einem nicht dargestellten Vorratsbehälter unmittelbar über die Einlassöffnung 22 in das Einwegeventil 1 und von dort über den geöffneten Freiraum 23 in die weitere Schlauchleitung gelangt, sodass ein schnelles Befüllen des gesamten Infusionsbesteckes in beschleunigter Form möglich ist. Nachdem sämtliche Schlauchleitungen befüllt sind kann durch manuelles Zusammendrücken der beiden Ventilgehäuseteile 2, 3 der Freiraum 23 verschlossen werden, sodass die Infusions- oder Nahrungsmittellösung nur noch über den Ventilkörper 20 seitlich in den Freiraum 23 fließen kann und von diesem über einen sich aufbauenden Druck infolge der einwirkenden Infusions- oder Schlauchpumpe auf die Flüssigkeit ein Öffnen der Dichtungslippe 28 gegenüber dem Ventilkörper 20 bewirkt. Hierzu kann in Flussrichtung vor dem Einwegeventil zusätzlich eine T- oder Y-Abzweigung vorgesehen werden, sodass beispielsweise eine Infusionspumpe anschließbar ist, die zeitweise ein Medikament zudosiert oder es wird zur Förderung einer Nahrungsmittellösung eine Schlauchpumpe eingesetzt. Somit gelangt die Flüssigkeit über den Freiraum 23 in die Auslassöffnung 26, wobei mit Hilfe weiterer nicht dargestellter Hilfsmittel, beispielsweise einer Schlauchklemme die Durchflussmenge eingestellt werden kann.

Durch den Einsatz des Einwegeventils 1 wird bei einem Rückstau aufgrund des höheren Druckes im unteren Bereich des Schlauchsystems die Dichtungslippe 28 der Ventildichtung 16 an den Ventilkörper 20 angepresst, wobei der Druck höher als der durch die Infusionspumpe erzeugte Druck sein kann, sodass ein Rückfluss in den Vorratsbehälter verhindert wird. Erst dann, wenn der Förderdruck höher ist, kann sich das Ventilelement 16 wieder öffnen und die Infusions- oder Nahrungsmittellösung durch das Einwegeventil 1 zum Patienten weiterfließen.

Das erfindungsgemäße Einwegeventil 1 ermöglicht somit einerseits ein schnelles Befüllen des Leitungssystem, beispielsweise bei einer Magensonde oder einer Injektionsnadel, kann aber ebenso nach manuellem Verschluss, dass heißt durch Zusammendrücken der beiden Ventilgehäuseteile 2, 3 als normales Einwegeventil eingesetzt werden, sodass infolge eines unterhalb des Einwegeventils 1 entstehenden Überdruckes ein Rückfluss in den Infusions- oder Nahrungsmittelbehälter ausgeschlossen werden kann. Im Wesentlichen besteht die Wirkung der Infusions- oder Schlauch pumpe darin, einen entsprechenden Förderdruck aufzubauen, und zwar von dem Infusionsbeutel zum Einwegeventil hin, damit die Dichtungslippe von dem Dichtungskörper aufgrund des Förderdruckes abheben kann und die Infusions- oder Nahrungsflüssigkeit über das Einwegeventil zum Patienten gelangt. Sollte ein Rückstau unterhalb des Einwegeventils entstehen, wird durch die Funktion des Einwegeventils verhindert, dass die Infusionslösung in Richtung auf den Infusionsbeutel gedrückt werden kann.

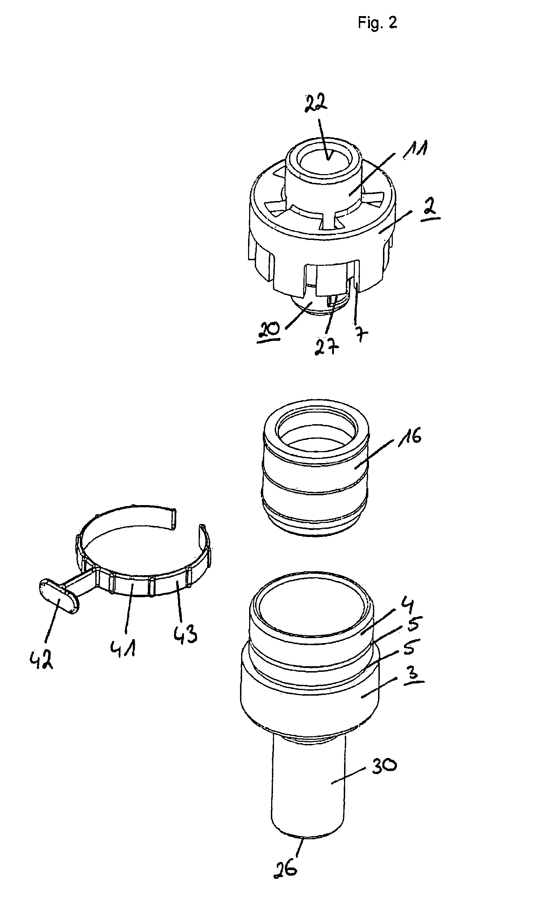

Figur 2 zeigt das erfindungsgemäße Einwegeventil 1 in einer perspektivischen Explosionsansicht, bestehend aus einem ersten Ventilgehäuseteil 2 mit Stutzen 11 und einem zweiten Ventilgehäuseteil 3 mit Stutzen 30. Der Stutzen 11 besitzt die Einlassöffnung 22, während der Stutzen 30 die Auslassöffnung 26 aufweist. Der Ventilkörper 16 ist ebenfalls rotationssymmetrisch ausgebildet und wird zunächst in die Eindrehung 18 bei der Montage in das zweite Ventilgehäuseteil 3 eingedrückt, um anschließend das erste Ventilgehäuseteil 2 unter Anlage der Dichtlippen 17 in die Ventildichtung 16 einzupressen, bis die Rastnasen 7 in die vorhandenen Raststufen 5 des zweiten Ventilgehäuseteils 3 einrasten. Hierbei ist vorgesehen, dass zunächst eine Einrastung in der ersten Raststufe 5 erfolgt, sodass eine große Durchflussöffnung gemäß der Beschreibung zu Figur 1 vorliegt. Bevor die beiden Ventilgehäuseteile 2, 3 zusammengeführt werden, wird ein Sicherungselement 41 mit einem Griffstück 42 und einem nicht geschlossenen Kreisringabschnitt 43 auf das zweite Ventilgehäuseteil 3 aufgesetzt, sodass beispielsweise die untere Raststufe 5 abgedeckt ist. Somit kann das Ventilgehäuseteil 2 nur bis zur ersten Raststufe 5 in die Ventildichtung 16 eingedrückt werden. Gleichzeitig ist hierdurch sichergestellt, dass eine großvolumige Verbindung zwischen Einlassöffnung 22 und Auslassöffnung 26 zur Erstbefüllung des Schlauchsystems besteht. Nachdem das Schlauchsystem gefüllt wurde, kann das Sicherungselement 41 durch Abziehen entfernt werden und durch anschließendes Zusammendrücken der beiden Ventilgehäuseteile 2, 3 erfolgt gemäß der vorliegenden Beschreibung zur Figur 1 eine Anlage der Dichtungslippe 28 an den Ventilkörper 20, um die Ventilfunktion des Rücklaufventils 1 einerseits zu gewährleisten und andererseits einen Flüssigkeitstransport von der Einlassöffnung 22 zur Auslassöffnung 26 infolge eines Druckaufbaus mit einem Abheben der Dichtungslippe 28 von dem Ventilkörper 20 zu ermöglichen.

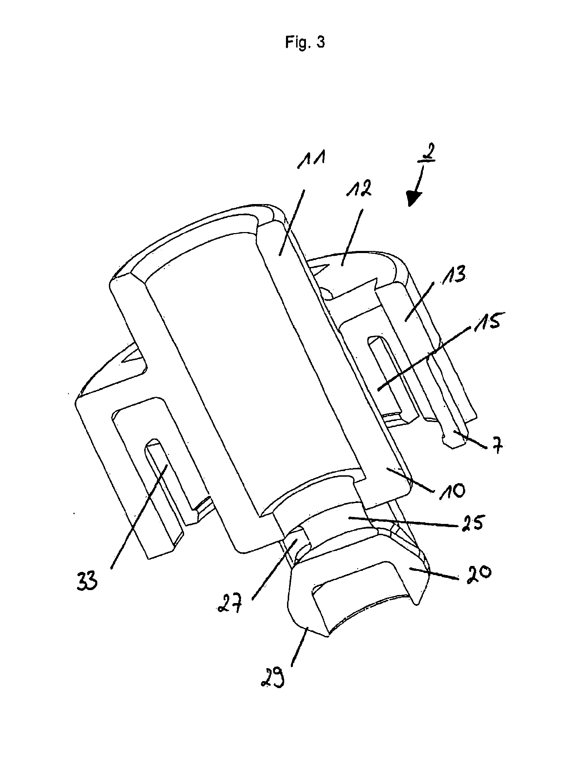

Figur 3 zeigt in einer perspektivischen geschnittenen Seitenansicht das erste Ventilgehäuseteil 2 mit einem innen liegenden Gehäuseteil 10 und einem außen liegenden Gehäusering 13 sowie einem Ventilkörper 20. Der Ventilkörper 20 ist einstückig mit dem Ventilgehäuseteil 2 verbunden und zwar über Stege 25, welche umfangsverteilt angeordnet sind, sodass zwischen den einzelnen Stegen 25 Öffnungen 27 verbleiben. Der Ventilkörper 20 ist hierbei rotationssymmetrisch aufgebaut und weist auf der Unterseite eine konische Anlagefläche 29 auf. Die Anlagefläche 29 dient zur Anlage der Dichtungslippe 28 der Ventildichtung 16. Das Gehäuseteil 20 endet oberhalb des Ventilgehäuseteils 2 in einem Stutzen 11, welcher zum Aufschieben eines Schlauchsystems verwendet werden kann. Das Gehäuseteil 10 und der Gehäusering 13 sind hierbei über Stege 12 einstückig miteinander verbunden, wobei die Stege 12 ebenfalls umfangsverteilt angeordnet sind. Zwischen Gehäuseteil 10 und Gehäusering 13 ist eine Ausnehmung 15 ausgebildet, welche zur Aufnahme des zweiten Ventilgehäuseteils 3 und der Ventildichtung 16 vorgesehen sind. Der Kreisring 13 weist im Weiteren umfangsverteilte Rastnasen 7 auf, welche durch schlitzförmige Ausnehmungen 33 im Gehäusering 13 ausgebildet sind. Somit wird die Elastizität der einzelnen Rastnasen 7 ausreichend gewährleistet.

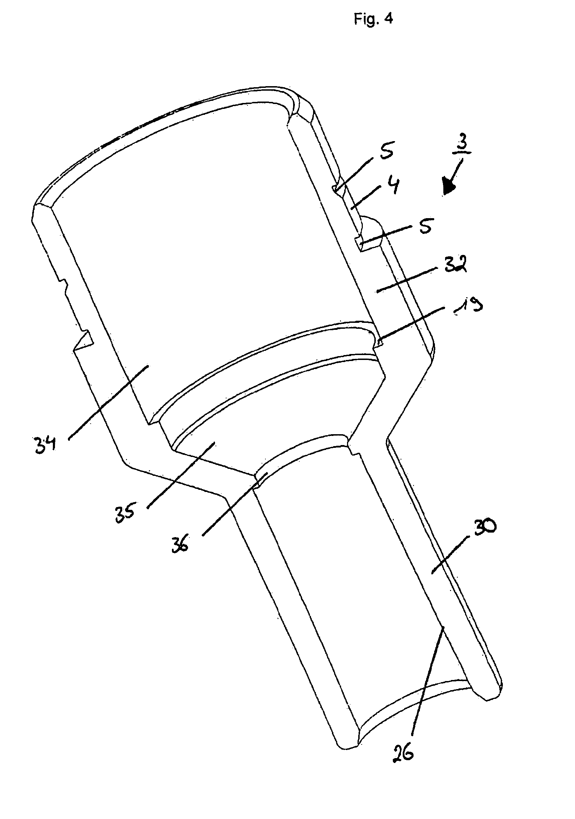

Figur 4 zeigt in einer perspektivischen geschnittenen Ansicht das zweite Ventilgehäuseteil 3, welches hohlzylindrisch ausgebildet aus einem Stutzen 30 mit Auslassöffnung 26 und einem Gehäusering 32 besteht. Auf der Außenfläche 4 des Gehäuserings 32 sind zwei Raststufen 5 ausgebildet, welche zur Aufnahme der Rastnasen 7 des ersten Ventilgehäuseteils 2 vorgesehen sind. Eine Anlageschulter 19 dient dazu, die eingelegte Ventildichtung 16 axial abzustützen, während die Ventildichtung 16 zusätzlich an der inneren Ringfläche 34 zu liegen kommt. Der Übergang vom Gehäusering 32 in den Stutzen 30 ist hierbei durch eine konisch verlaufende Fläche 35 gewährleistet, welche über eine Öffnung 36 unmittelbar in die Auslassöffnung 26 mündet.

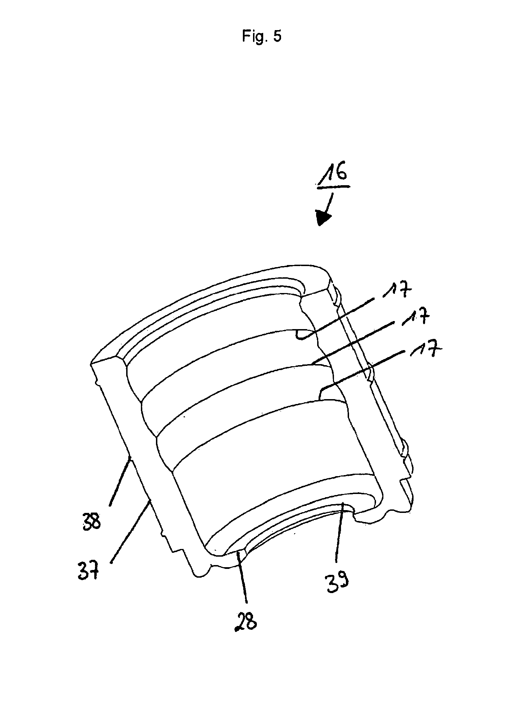

Figur 5 zeigt in einer geschnittenen perspektivischen Ansicht die Ventildichtung 16, welche aus einem elastischen Material besteht und ebenfalls ringförmig ausgebildet ist. Auf der Innenfläche sind mehrere Dichtungslippen 17 vorgesehen, welche unmittelbar zur Anlage an dem Gehäuseteil 10 des ersten Ventilgehäuseteils 2 vorgesehen sind.

Auf der Außenfläche 37 befinden sich mehrere Dichtungsringe 38, welche zur Anlage an den Gehäusering 32 des zweiten Ventilgehäuseteils 3 vorgesehen sind. Die Ventildichtung 16 weist im Weiteren eine kreisringförmige Öffnung 39 auf, welche in eine Dichtungslippe 28 übergeht. Die Dichtungslippe 28 dient zur Anlage an den Ventilkörper 20 des ersten Ventilgehäuseteils 2.

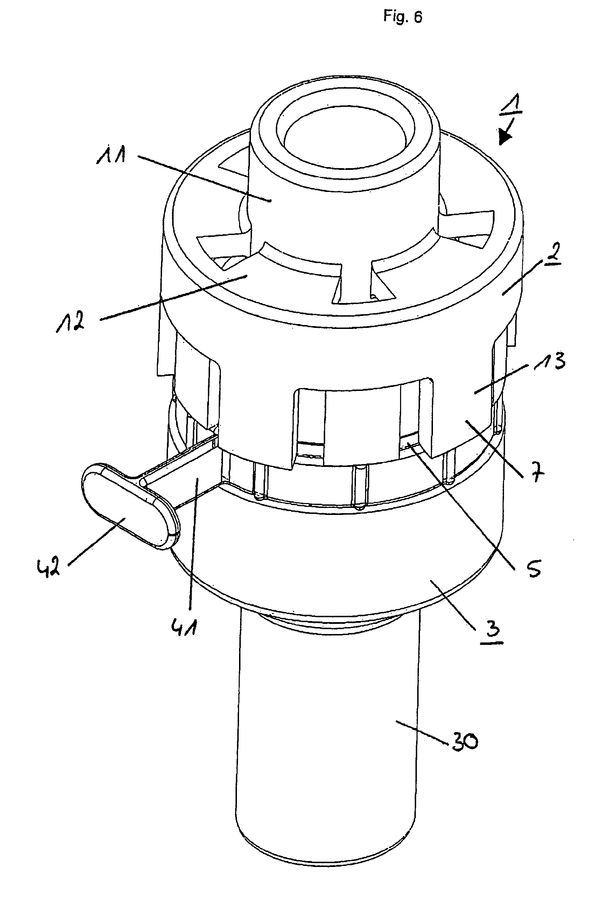

Figur 6 zeigt in einer perspektivischen Seitenansicht das erfindungsgemäße Einwegeventil 1 nach erfolgten Zusammenbau, und zwar bestehend aus dem ersten Ventilgehäuseteil 2 sowie dem zweiten Ventilgehäuseteil 3. Zwischen beiden Ventilgehäuseteilen 2, 3 ist das Sicherungselement 41 mit Griffstück 42 angeordnet, sodass nach Abziehen des Sicherungselementes 41 ein Zusammendrücken der beiden Ventilgehäuseteile 2, 3 ermöglicht wird. Das Einwegeventil 1 kann hierbei über den oberen Anschlussstutzen 11 sowie dem unteren Anschlussstutzen 30 mit dem Schlauchsystem verbunden werden. Aus dieser Ansicht ist darüber hinaus nochmals zu erkennen, dass der Anschlussstutzen 11 über umfangsverteilte Stege 12 mit dem Gehäusering 13 verbunden ist. Ebenso deutlich ist zu erkennen, wie die Haltekrallen 7 in der ringförmigen Raststufe 5 des weiteren Ventilgehäuseteils 3 einliegen.

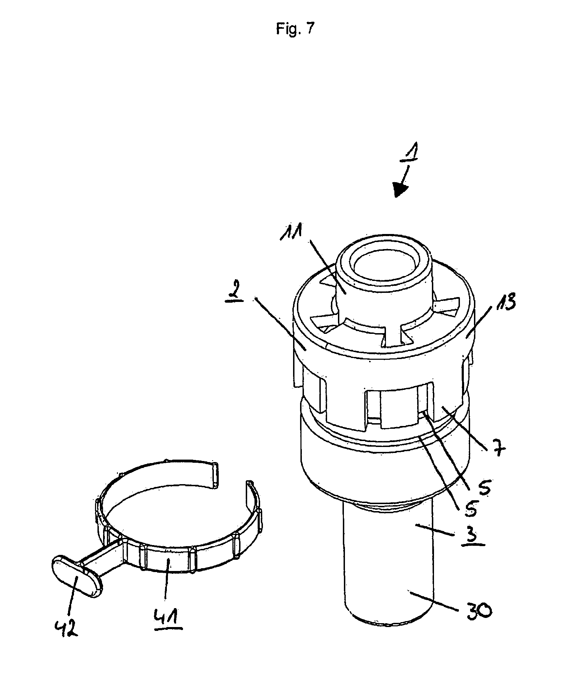

Figur 7 zeigt das Einwegeventil 1 gemäß Figur 6 nachdem das Sicherungselement 41 entfernt worden ist. Die beiden Ventilgehäuseteile 2, 3 sind bei dieser Darstellung noch nicht zusammengedrückt, da sich die Rastnasen 7 in der oberen Raststufe 5 befinden.

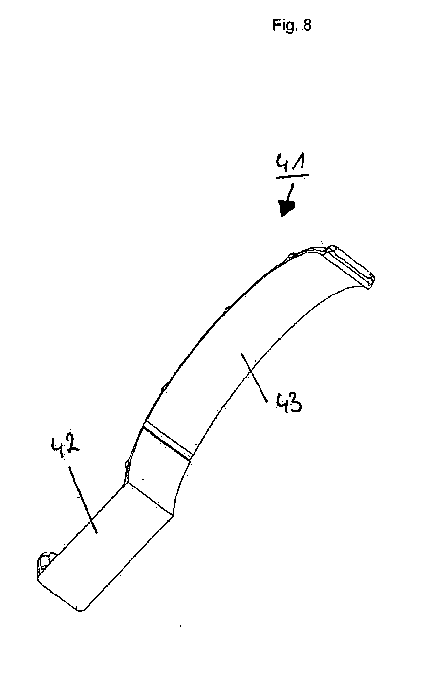

Figur 8 zeigt in einer geschnittenen perspektivischen Ansicht nochmals das Sicherungselement 41, bestehend aus einem Griffstück 42 und einem nicht vollständig geschlossenen Kreisringabschnitt 43. Die Breite des Sicherungselementes 41 ist hierbei an den Abstand der beiden Raststufen 5 des ersten Ventilgehäuseteils 2 angepasst. Ebenso wie die übrigen Teile des Einwegeventils 1 besteht das Sicherungselement 41 aus einem elastischen Kunststoffteil, sodass das Sicherungselement 41 ohne Probleme von dem Einwegeventil 1 abgezogen werden kann.

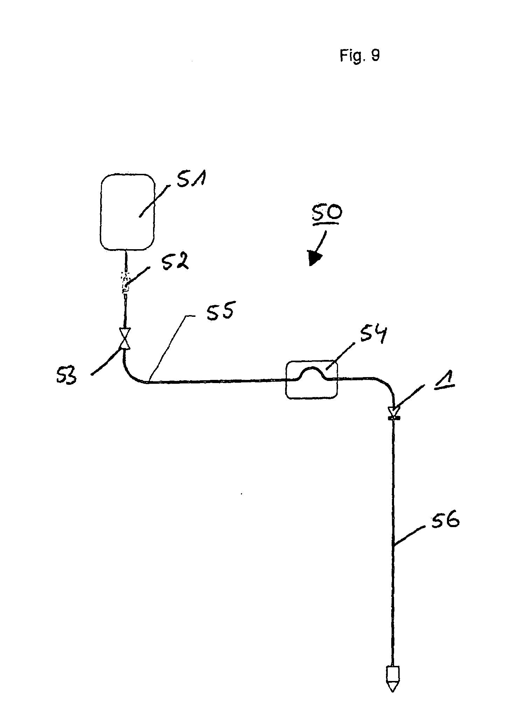

Figur 9 zeigt in einer schematischen Ansicht ein Infusionsbesteck 50, welches aus einem Infusionsbeutel 51 mit Anschlussverbindung 52 und einer Schlauchklemme 53 einerseits und einer Schlauchpumpe 54 sowie einem erfindungsgemäßen Einwegeventil 1 andererseits besteht. Eine Schlauchverbindung 55 führt hierbei zunächst von dem Infusionsbeuteil 51 ausgehend über die Anschlussverbindung 52 und die Schlauchklemme 53 zur Schlauchpumpe 54. Die Schlauchpumpe 54 kann im einfachsten Fall mit Hilfe eines Flügelrades eine Kraft auf die elastische Schlauchverbindung 55 ausüben, sodass ein Förderdruck der Infusionslösung entsteht. Dieser Förderdruck reicht bereits aus, um die Dichtungslippe des Einwegeventils 1 von dem Dichtungskörper abzuheben, sodass die Infusionslösung über einen Schlauch 56 dem Patienten zugeführt werden kann.

### Bezugszeichenliste

- 1: Einwegeventil
- 2: Ventilgehäuseteil
- 3: Ventilgehäuseteil
- 4: Außenfläche
- 5: Raststufe
- 7: Rastnase
- 10: Gehäuseteil
- 11: Stutzen
- 12: Verbindungsstrebe
- 13: Gehäusering
- 14: Stirnfläche
- 15: Ausnehmung
- 16: Ventildichtung
- 17: Dichtlippe
- 18: Eindrehung
- 19: Anlageschulter
- 20: Ventilkörper
- 21: Durchlassöffnung
- 22: Einlassöffnung
- 23: Freiraum
- 24: Eintrittsöffnung
- 25: Steg
- 26: Auslassöffnung
- 27: Öffnung
- 28: Dichtungslippe
- 29: Anlagefläche
- 30: Stutzen
- 32: Gehäusering
- 40: Einwegeventil
- 41: Sicherungselement
- 42: Griffstück
- 43: Kreisringabschnitt
- 50: Infusionsbesteck
- 51: Infusionsbeutel
- 52: Anschlussverbindung
- 53: Schlauchklemme
- 54: Schlauchpumpe
- 55: Schlauchverbindung
- 56: Schlauch

## Patentansprüche

1. Einwegeventil (1) für medizinische Anwendung, umfassend zumindest ein Ventilgehäuse, eine Einlass- (22) und Auslassöffnung (26) und eine Ventilanordnung, welche aus einem Ventilkörper (20) und einer Ventildichtung (16) besteht,
**dadurch gekennzeichnet, dass**
der Ventilkörper (20) eine Durchlassöffnung (21) aufweist, welche in einem abdichtbaren Freiraum (23) zwischen dem Ventilkörper (20) und der Ventildichtung (16) mündet, und dass
die Ventildichtung (16) eine Öffnung (39) aufweist, welche durch den Ventilkörper (20) bei einer Relativbewegung zweier Gehäuseteile (2, 3) derart verschließbar ist, dass die Ventildichtung (16) mit einer Dichtungslippe (28) an dem Ventilkörper (20) elastisch anhebbar anliegt.

2. Einwegeventil (1) nach Anspruch 1, **dadurch gekennzeichnet, dass** das Ventilgehäuse aus zumindest zwei Ventilgehäuseteilen (2, 3) besteht, welche wenigstens teilweise ineinander beziehungsweise zusammenschiebbar ausgebildet sind.

3. Einwegeventil (1) nach einem der Ansprüche 1 bis 2, **dadurch gekennzeichnet, dass** die Ventilgehäuseteile (2, 3) rotationssymmetrisch und axial bauend ausgebildet sind oder dass die Ventilgehäuseteile (2, 3) rotationssymmetrisch und teilweise abgewinkelt ausgeführt sind.

4. Einwegeventil (1) nach einem der Ansprüche 1 bis 3, **dadurch gekennzeichnet, dass** die Ventilgehäuseteile (2, 3) eine Andruckfläche (12) und eine Gegenhalterfläche aufweisen.

5. Einwegeventil (1) nach einem der Ansprüche 1 bis 4, **dadurch gekennzeichnet, dass** ein Ventilgehäuseteil (2, 3) ein oder mehrere Raststufen (5) auf einer Außenfläche (4) aufweist und dass das korrespondierende Ventilgehäuseteil (2, 3) eine oder mehrere umfangsverteilte Rastnasen (7) aufweist.

6. Einwegeventil (1) nach einem der Ansprüche 1 bis 5, **dadurch gekennzeichnet, dass** die Rastnasen (7) des Ventilgehäuseteils (2, 3) durch ein entfernbares Sicherungselement (41) sperrbar sind.

7. Einwegeventil (1) nach einem der Ansprüche 1 bis 6, **dadurch gekennzeichnet, dass** das Sicherungselement (41) aus einem Kreisringsegment mit Griffstück (42) besteht.

8. Einwegeventil (1) nach einem der Ansprüche 1 bis 7, **dadurch gekennzeichnet, dass** zwischen den Ventilgehäuseteilen (2, 3) eine Ventildichtung (16) angeordnet ist, welche in einer Eindrehung des ersten Ventilgehäuseteils (2, 3) einliegt und durch eine Anlageschulter (19) gehalten wird und mit zumindest einer ersten Dichtlippe (17) an dem zweiten Ventilgehäuseteil (2, 3) abdichtend und mit einer weiteren Dichtungslippe (28) an dem Ventilkörper (20) anliegt.

9. Einwegeventil (1) nach einem der Ansprüche 1 bis 8, **dadurch gekennzeichnet, dass** die Ventildichtung (16) mehrere nach innen weisende Dichtungslippen (17) aufweist, welche an einer Axialfläche eines Ventilgehäuseteils (2, 3) anpressbar anliegen.

10. Einwegeventil (1) nach einem der Ansprüche 1 bis 9, **dadurch gekennzeichnet, dass** die Ventildichtung (16) rotationssymmetrisch ausgebildet eine nach innen weisende zweite Dichtungslippe (19) aufweist, welche am Ventilkörper (20) anliegt.

11. Einwegeventil (1) nach einem der Ansprüche 1 bis 10, **dadurch gekennzeichnet, dass** der Ventilkörper (20) rotationssymmetrisch ausgebildet und einstückig mit einem Ventilgehäuseteil (2, 3) verbunden ist.

12. Einwegeventil (1) nach einem der Ansprüche 1 bis 11, **dadurch gekennzeichnet, dass** der Ventilkörper (20) eine konische Anlagefläche (29) für die Ventildichtung (16) aufweist.

13. Einwegeventil (1) nach einem der Ansprüche 1 bis 12, **dadurch gekennzeichnet, dass** die Dichtungslippe (28) linienförmig an dem Ventilkörper (20) anliegt.

14. Einwegeventil (1) nach einem der Ansprüche 1 bis 13, **dadurch gekennzeichnet, dass** eine Einlassöffnung (22) in einen Freiraum (23) mündet und eine Auslassöffnung (26) unterhalb der Ventildichtung mündet.

15. Einwegeventil (1) nach einem der Ansprüche 1 bis 14, **dadurch gekennzeichnet, dass** die Strömungsrichtung ausgehend von der Einlassöffnung (22) über die Öffnung (27) des Ventilkörpers (20) bis zur anliegenden Dichtungslippe (28) führt und durch einen Druckaufbau ein Abheben der Dichtungslippe (28) vom Ventilkörper (20) eine Verbindung zur Auslassöffnung (26) herstellt.

16. Einwegeventil (1) nach einem der Ansprüche 1 bis 15, **dadurch gekennzeichnet, dass** die Dichtungslippe (28) eine Vorspannung aufweist, welche durch den Durchmesser des Ventilkörpers (20), die Dicke der Dichtungslippe (28), den Innendurchmesser der Dichtungslippe (28) oder durch die Shore-Härte bestimmbar ist.

17. Einwegeventil (1) nach einem der Ansprüche 1 bis 16, **dadurch gekennzeichnet, dass** die Dichtungslippe (28) bei einem Druckaufbau von etwa 20 bis etwa 300 kPascal von dem Ventilkörper (20) abhebt.

18. Einwegeventil (1) nach einem der Ansprüche 1 bis 17, **dadurch gekennzeichnet, dass** die Ventildichtung (16) eine Shore-Härte von 60 bis 80 SH-A aufweist.

## Claims

1. One-way valve (1) for medicinal use, comprises at least one valve housing, an inlet (22) and outlet opening (26) and a valve arrangement which comprises a valve body (20) and a valve seal (16), **characterised in that** the valve body (20) has a through-flow opening (21) which opens in a sealable expansion space (23) between the valve body (20) and the valve seal (16), and that the valve seal (16) has an opening (39) which can be closed by the valve body (20) during relative movement of two housing parts (2, 3) so that the valve seal (16) adjoins with a sealing lip (28) against the valve body (20) where it can be resiliently lifted.

2. One-way valve (1) according to claim 1 **characterised in that** the valve housing consists of at least two valve housing parts (2, 3) which are designed to slide into or up to one another at least in part.

3. One-way valve (1) according to one of claims 1 to 2 **characterised in that** the valve housing parts (2, 3) are designed to be built rotationally symmetrical and axially or that the valve housing parts (2, 3) are designed rotationally symmetrical and partially angled.

4. One-way valve (1) according to one of claims 1 to 3 **characterised in that** the valve housing parts (2, 3) have a contact pressure face (12) and a counter holder face.

5. One-way valve (1) according to one of claims 1 to 4 **characterised in that** a valve housing part (2, 3) has one or more detent steps (5) on an outside surface (4) and that the corresponding valve housing part (2, 3) has one or more circumferentially distributed detent lugs (7).

6. One-way valve (1) according to one of claims 1 to 5 **characterised in that** the detent lugs (7) of the valve housing part (2, 3) are lockable by a removable security element (41).

7. One-way valve (1) according to one of claims 1 to 6 **characterised in that** the security element (41) consists of a circular ring segment with grip member (42).

8. One-way valve (1) according to one of claims 1 to 7 **characterised in that** between the valve housing parts (2, 3) there is a valve seal (16) which is placed in a groove of the first valve housing part (2, 3) and is held by a bearing shoulder (19) and with at least one first sealing lip (17) bears sealingly against the second valve housing part (2, 3) and with a further sealing lip (28) bears against the valve body (20).

9. One-way valve (1) according to one of claims 1 to 8 **characterised in that** the valve seal (16) has several inward facing sealing lips (17) which adjoin and can be pressed against an axial face of a valve housing part (2,3).

10. One-way valve (1) according to one of claims 1 to 9 **characterised in that** the valve seal (16) has formed rotationally symmetrical an inward facing second sealing lip (19) which bears against the valve body (20).

11. One-way valve (1) according to one of claims 1 to 10 **characterised in that** the valve body (20) is formed rotationally symmetrical and is connected integral with a valve housing part (2, 3).

12. One-way valve (1) according to one of claims 1 to 11 **characterised in that** the valve body (20) has a conical bearing face (29) for the valve seal (16).

13. One-way valve (1) according to one of claims 1 to 12 **characterised in that** the sealing lip (28) bears linearly against the valve body (20).

14. One-way valve (1) according to one of claims 1 to 13 **characterised in that** an inlet opening (22) opens into an expansion space (23) and an outlet opening (26) opens underneath the valve seal.

15. One-way valve (1) according to one of claims 1 to 14 **characterised in that** the flow direction starting from the inlet opening (22) leads through the opening (27) of the valve body (20) up to the adjoining sealing lip (28) and through a build-up of pressure a lift of the sealing lip (28) from the valve body (20) produces a connection to the outlet opening (26).

16. One-way valve (1) according to one of claims 1 to 15 **characterised in that** the sealing lip (28) has a pretension which can be determined by the diameter of the valve body (20), the thickness of the sealing lip (28), the internal diameter of the sealing lip (28) or by the Shore hardness.

17. One-way valve (1) according to one of claims 1 to 16 **characterised in that** the sealing lip (28) is lifted from the valve body (20) with a build-up of pressure from about 20 to about 300 kPascal.

18. One-way valve (1) according to one of claims 1 to 17 **characterised in that** the valve seal (16) has a Shore hardness of 60 to 80 SH-A.

## Revendications

1. Vanne unidirectionnelle (1) pour application médicale, comprenant au moins un boîtier de vanne, une ouverture d'entrée (22) et de sortie (26) et un agencement de vanne qui se compose d'un corps de vanne (20) et d'une garniture de vanne (16),
**caractérisée en ce que**
le corps de vanne (20) présente une ouverture de passage (21) qui débouche dans un espace libre (23) pouvant être rendu étanche entre le corps de vanne (20) et la garniture de vanne (16), et **en ce que**
la garniture de vanne (16) présente une ouverture (39) qui peut être fermée par le corps de vanne (20) lors d'un mouvement relatif de deux parties de boîtier (2, 3) de telle manière que la garniture de vanne (16) repose de manière relevable élastiquement avec une lèvre d'étanchéité (28) contre le corps de vanne (20).

2. Vanne unidirectionnelle (1) selon la revendication 1, **caractérisée en ce que** le boîtier de vanne se compose d'au moins deux parties de boîtier de vanne (2, 3) qui sont réalisées au moins en partie l'une dans l'autre ou de manière coulissante.

3. Vanne unidirectionnelle (1) selon l'une des revendications 1 à 2, **caractérisée en ce que** les parties de boîtier de vanne (2, 3) sont réalisées de manière symétrique en rotation et s'élevant axialement ou **en ce que** les parties de boîtier de vanne (2, 3) sont réalisées de manière symétrique en rotation et partiellement coudée.

4. Vanne unidirectionnelle (1) selon l'une des revendications 1 à 3, **caractérisée en ce que** les parties de boîtier de vanne (2, 3) présentent une surface de pression (12) et une surface de contre-support.

5. Vanne unidirectionnelle (1) selon l'une des revendications 1 à 4, **caractérisée en ce qu'**une partie de boîtier de vanne (2, 3) présente un ou plusieurs étages d'encliquetage (5) sur une surface extérieure (4) et **en ce que** la partie de boîtier de vanne (2, 3) correspondante présente un ou plusieurs nez d'encliquetage (7) répartis sur la périphérie.

6. Vanne unidirectionnelle (1) selon l'une des revendications 1 à 5, **caractérisée en ce que** les nez d'encliquetage (7) de la partie de boîtier de vanne (2, 3) peuvent être bloqués par un élément de blocage (41) amovible.

7. Vanne unidirectionnelle (1) selon l'une des revendications 1 à 6, **caractérisée en ce que** l'élément de blocage (41) se compose d'un segment annulaire et circulaire avec une pièce de préhension (42).

8. Vanne unidirectionnelle (1) selon l'une des revendications 1 à 7, **caractérisée en ce qu'**une garniture de vanne (16) est agencée entre les parties de boîtier de vanne (2, 3), laquelle se trouve dans une gorge tournée de la première partie de boîtier de vanne (2, 3) et est maintenue par un épaulement d'appui (19) et repose de manière étanche avec au moins une première lèvre d'étanchéité (17) contre la seconde partie de boîtier de vanne (2, 3) et avec une autre lèvre d'étanchéité (28) contre le corps de vanne (20).

9. Vanne unidirectionnelle (1) selon l'une des revendications 1 à 8, **caractérisée en ce que** la garniture de vanne (16) présente plusieurs lèvres d'étanchéité (17) dirigées vers l'intérieur qui reposent de manière à pouvoir être pressés contre une surface axiale d'une partie de boîtier de vanne (2, 3).

10. Vanne unidirectionnelle (1) selon l'une des revendications 1 à 9, **caractérisée en ce que** la garniture de vanne (16) présente, réalisée de manière symétrique en rotation, une seconde lèvre d'étanchéité (19) dirigée vers l'intérieur qui repose contre le corps de vanne (20).

11. Vanne unidirectionnelle (1) selon l'une des revendications 1 à 10, **caractérisée en ce que** le corps de vanne (20) est réalisé de manière symétrique en rotation et relié d'un seul tenant à une partie de boîtier de vanne (2, 3).

12. Vanne unidirectionnelle (1) selon l'une des revendications 1 à 11, **caractérisée en ce que** le corps de vanne (20) présente une surface d'appui conique (29) pour la garniture de vanne (16).

13. Vanne unidirectionnelle (1) selon l'une quelconque des revendications 1 à 12, **caractérisée en ce que** la lèvre d'étanchéité (28) repose de manière linéaire contre le corps de vanne (20).

14. Vanne unidirectionnelle (1) selon l'une des revendications 1 à 13, **caractérisée en ce qu'**une ouverture d'entrée (22) débouche dans un espace libre (23) et une ouverture de sortie (26) débouche sous la garniture de vanne.

15. Vanne unidirectionnelle (1) selon l'une des revendications 1 à 14, **caractérisée en ce que** le sens d'écoulement mène de l'ouverture d'entrée (22) en passant par l'ouverture (27) du corps de vanne (20) jusqu'à la lèvre d'étanchéité fermée (28) et par un établissement de pression, un levage de la lèvre d'étanchéité (28) du corps de vanne (20) établit une liaison avec l'ouverture de sortie (26).

16. Vanne unidirectionnelle (1) selon l'une des revendications 1 à 15, **caractérisée en ce que** la lèvre d'étanchéité (28) présente une précontrainte qui peut être déterminée par le diamètre du corps de vanne (20), l'épaisseur de la lèvre d'étanchéité (28), le diamètre intérieur de la lèvre d'étanchéité (28) ou par la dureté Shore.

17. Vanne unidirectionnelle (1) selon l'une des revendications 1 à 16, **caractérisée en ce que** la lèvre d'étanchéité (28) se lève du corps de vanne lors d'un établissement de pression d'environ 20 à environ 300 kPa.

18. Vanne unidirectionnelle (1) selon l'une des revendications 1 à 17, **caractérisée en ce que** la garniture de vanne (16) présente une dureté Shore de 60 à 80 SH-A.
